# EUROPEAN PATENT APPLICATION

(11) **EP 4 328 284 A1**
(43) Date of publication of application: **28.02.2024**
(21) Application number: 22791558.4
(22) Date of filing: 30.03.2022
(51) Int. Cl.: C09K 5/14, A61B 5/055, F25B 9/00, G01N 24/00, H01F 6/00

(54) **MAGNETIC COLD STORAGE MATERIAL PARTICLE, COLD STORAGE DEVICE, REFRIGERATING MACHINE, CRYOPUMP, SUPERCONDUCTING MAGNET, NUCLEAR MAGNETIC RESONANCE IMAGING APPARATUS, NUCLEAR MAGNETIC RESONANCE APPARATUS, MAGNETIC-FIELD-APPLICATION-TYPE SINGLE CRYSTAL PULLING APPARATUS, AND HELIUM RE-CONDENSATION APPARATUS**

(30) Priority: 20.04.2021 JP 2021070760
(71) Applicant: Kabushiki Kaisha Toshiba, Tokyo 105-0023 (JP); Toshiba Materials Co., Ltd., Isogo-Ku Yokohama-Shi Kanagawa 235-0032 (JP)
(72) Inventor: KAWAMOTO, Takahiro, Yokohama-shi, Kanagawa 235-0032 (JP); USUI, Daichi, Yokohama-shi, Kanagawa 235-0032 (JP); HIRAMATSU, Ryosuke, Yokohama-shi, Kanagawa 235-0032 (JP)
(74) Representative: Henkel & Partner mbB
(86) International application number: PCT/JP2022/016240
(87) International publication number: WO 2022/224783

(57) **Abstract**

Magnetic cold storage material particles with a low breakage rate in the case of being subjected to long-term vibration caused by operation of a refrigerator under a cryogenic temperature are provided. A cold storage device and a refrigerator, each of which includes the above-described magnetic cold storage material particles and does not degrade refrigeration performance under long-term operation, are provided. Apparatuses provided with this refrigerator, such as a superconducting magnet, are provided.

Each magnetic cold storage material particle of the embodiment is composed of an intermetallic compound containing a rare earth element, and an area percentage of voids present in its cross-section is 0.0001% or more and 15% or less. Each of the cold storage device of the embodiment, the refrigerator of the embodiment, and the apparatuses provided with this refrigerator, such as a superconducting magnet, includes the magnetic cold storage material particles of the embodiment.

## Description

### TECHNICAL FIELD

Embodiments of the present invention relate to magnetic cold storage material particles, a cold storage device, a refrigerator, a cryopump, a superconducting magnet, an MRI (magnetic resonance imaging) apparatus, a nuclear magnetic resonance apparatus, a magnetic-field-application-type single-crystal puller, and a helium re-condensation apparatus.

### BACKGROUND

In recent years, superconducting technology has been remarkably advanced, and development of a high-performance and highly-reliable cryogenic refrigerator has become essential with the expansion of application fields of the superconducting technology. Such a cryogenic refrigerator is required to exhibit high thermal efficiency for a long period of time.

In the cryogenic refrigerator, a plurality of cold storage materials are accommodated in a cold storage device. For example, cold is generated by heat exchange between the cold storage materials and helium gas passing through the cold storage device. For example, in a superconducting MRI apparatus and/or a cryopump, a refrigerator using a refrigeration cycle such as a Gifford-McMahon (GM) system, a Stirling system, or a pulse tube system is used.

In addition, a high-performance refrigerator is required for a magnetically levitated train in order to generate magnetic force by using a superconducting magnet. Recently, the high-performance refrigerator has also been used in a superconducting magnetic energy storage (SMES), a magnetic-field-application type single-crystal puller configured to produce high-quality silicon wafers, and the like. Development and practical application of a pulse tube refrigerator, which is expected to have higher reliability, are also actively promoted.

In the superconducting magnet and/or the MRI apparatus described above, liquid helium to be used evaporates, so replenishment of the liquid helium poses a problem. In recent years, the problem of helium depletion has become more serious and it has become difficult to obtain helium, affecting the industrial world.

In order to reduce consumption amount of the liquid helium and reduce the burden of maintenance such as replenishment, a helium re-condensation apparatus configured to re-condense evaporated helium has been put into practical use, and its demand is increasing. In this helium re-condensation apparatus, the GM type refrigerator and/or the pulse tube type refrigerator are also used for cooling down the temperature to 4K level in order to liquefy the helium.

Additionally, in order to prevent liquefaction of gases excluding helium or prevent evaporation of liquefied gases during its storage, the GM type refrigerator and the pulse tube type refrigerator can be used. The gases excluding helium include, for example, hydrogen. Since the boiling point of liquid hydrogen is about 20K, evaporation of the liquid hydrogen can be reduced by keeping the temperature below 20K.

In a refrigerator provided with a cold storage material, a working medium such as compressed helium (He) gas flows unidirectionally in a cold storage device containing the cold storage material, and its heat energy is supplied to the cold storage material. The expanded working medium flows in the opposite direction in the cold storage device so as to receive thermal energy from the cold storage material. As the heat recuperation effect in these processes improves, the thermal efficiency in the working medium cycle increases and a lower temperature can be achieved.

As the specific heat per unit volume of the cold storage material installed in the cold storage device becomes higher, the cold storage material can store more thermal energy, which improves the refrigeration capacity of the refrigerator. Thus, it is preferred that: a cold storage material exhibiting a high specific heat at a low temperature is provided on the low temperature side of the cold storage device; and another cold storage material exhibiting a high specific heat at a high temperature is provided on the high temperature side of the cold storage device.

A cold storage material exhibits a high volumetric specific heat in a specific temperature range depending on its composition. Hence, the cold storage capacity is enhanced by combining cold storage materials that are different in composition and exhibit a high volumetric specific heat in the target temperature range, and thereby the refrigeration capacity of the refrigerator is improved.

In the conventional refrigerators, refrigeration at 4K has been achieved by combining: a metallic cold storage material such as lead (Pb), bismuth (Bi), and tin (Sn) on the high temperature side; and a metallic magnetic cold storage material such as Er₃Ni, ErNi, and HoCu₂ on the low temperature side of 20K or less. In addition, a non-metallic magnetic cold storage material such as Gd₂O₂S is used in combination with the above-described metallic magnetic cold storage material in some cases.

In recent years, superconducting apparatuses using high-temperature superconducting wires have also been developed. These apparatuses are being considered for operation at 10K to 30K. For refrigerators operating at these temperatures, it is preferred to use a cold storage material that exhibits a high volumetric specific heat at 10K to 30K.

In the case of applying a refrigerator to a superconducting apparatus and prevention of evaporation of liquefied gas, the refrigerator is required to exhibit a high thermal efficiency for a long period of time. The cold storage material is subjected to mechanical vibration during the heat exchange cycle between the cold storage material and a working substance. Thus, during operation of the refrigerator, the cold storage material is constantly subjected to vibration at the operating temperature of the refrigerator. If the amount of the cold storage material damaged by this vibration reaches a certain amount, the flow of the working medium such as helium (He) gas deteriorates and thereby the thermal efficiency decreases. If the cold storage material is severely damaged, the flow of the working medium such as He gas stops, and consequently, the refrigerator stops.

Hence, in order to improve long-term reliability of the refrigerator, the cold storage material is required to suppress its breakage amount to a certain amount or less in the case of being subjected to vibration for a long time at the operating temperature of the refrigerator. For example, the operating temperature of the refrigerator is: liquid helium temperature (about 4K) for MRI using low-temperature superconducting wires; about 10K to 30K for an apparatus using high-temperature superconducting wires; and liquid hydrogen temperature (about 20K) for cooling down liquid hydrogen.

### PRIOR ART DOCUMENT

### PATENT DOCUMENT

[Patent Document 1] JP 2003-073661 A
[Patent Document 2] WO 1996/006315 A1
[Patent Document 3] WO 2014/057657 A1

### SUMMARY

### PROBLEMS TO BE SOLVED BY INVENTION

An object of the present invention is to provide: magnetic cold storage material particles that have a low breakage rate in the case of being subjected to long-term vibration at a cryogenic temperature; and a cold storage device that includes the above-described magnetic cold storage material particles and thereby does not degrade its refrigeration performance even in the case of being operated for a long period of time.

### SOLUTION TO PROBLEM

A magnetic cold storage material particle of one embodiment is composed of an intermetallic compound containing a rare earth element represented by RMz, wherein: R is at least one rare earth element selected from Y, La, Ce, Pr, Nd, Pm, Sm, Eu, Gd, Tb, Dy, Ho, Er, Tm, and Yb; M is at least one metal element selected from Ni, Co, Cu, Ag, Si, Ga, Bi, Al, and Ru; and z is number in a range from 0.001 to 9.0, wherein an area percentage of voids present in a cross-section of the magnetic cold storage material particle is 0.0001% or more and 15% or less.

A cold storage device according to one embodiment is provided with magnetic cold storage material particles, each of which contains a rare earth element represented by RMz, wherein 70% or more of these magnetic cold storage material particles have a void area percentage (i.e., area percentage of voids present in a cross-section of each magnetic cold storage material particle) of 0.0001% or more and 15% or less.

### BRIEF DESCRIPTION OF DRAWINGS

Fig. 1 is a schematic cross-sectional view illustrating magnetic cold storage material particles according to the first embodiment and a main configuration of a refrigerator according to the third embodiment.
Fig. 2 is a cross-sectional view illustrating an overall configuration of a cryopump according to the fourth embodiment.
Fig. 3 is a perspective view illustrating an overall configuration of a superconducting magnet according to the fifth embodiment.
Fig. 4 is a cross-sectional view illustrating an overall configuration of an MRI apparatus according to the sixth embodiment.
Fig. 5 is a cross-sectional view illustrating an overall configuration of a nuclear magnetic resonance apparatus according to the seventh embodiment.
Fig. 6 is a perspective view illustrating an overall configuration of a magnetic-field-application-type single-crystal puller according to the eighth embodiment.
Fig. 7 is a perspective view illustrating an overall configuration of a helium re-condensation apparatus according to the ninth embodiment.

### DETAILED DESCRIPTION

Hereinbelow, embodiments of the present invention will be described by referring to the accompanying drawings. In the following description, the same or similar components are denoted by the same reference signs, and description of the components having already been described is omitted as appropriate.

In the present specification, a cryogenic temperature means a temperature range of 30K or less, for example. In the temperature range of 30K or less, a superconducting apparatus can operate and liquefaction of helium or hydrogen can be achieved.

### (First Embodiment)

Each magnetic cold storage material particle according to the first embodiment is composed of an intermetallic compound containing a rare earth element represented by RMz, wherein: R is at least one rare earth element selected from Y, La, Ce, Pr, Nd, Pm, Sm, Eu, Gd, Tb, Dy, Ho, Er, Tm, and Yb; M is at least one metal element selected from Ni, Co, Cu, Ga, Bi, Ag, Si, Al, and Ru; and z is number in a range from 0.001 to 9.0.

In the magnetic cold storage material particles according to the first embodiment, an area percentage of voids present in a cross-section of each magnetic cold storage material particle is 0.0001% or more and 15% or less.

The cross-sectional area of the magnetic cold storage material particle of the first embodiment and the area occupied by voids present in the cross-section can be obtained by image analysis of an optical microscope image or a scanning electron microscope (SEM) image. In the image analysis of the SEM image, a backscattered-electron image is used. For example, ImageJ can be used as image analysis software.

On the basis of brightness of the backscattered electron image, both the regions corresponding to the voids and the regions corresponding to non-voids in the cold storage material particle can be extracted from the image. At the time of extracting the corresponding regions, for example, brightness is binarized. For example, when the extracted region and the original image are compared with each other by the naked eye and it is clear that the voids are not properly extracted, the binarization conditions are adjusted, and then the particle cross-section and the area of voids are evaluated.

In the case of observing the cross-section of each magnetic cold storage material particle according to the first embodiment, it is preferred to use a sample, cross-section of which is exposed by: impregnating the magnetic cold storage material particles in resin; and then polishing them with an ion milling apparatus. At this time, when polishing is performed by using abrasive paper, weak portions in terms of strength in the cold storage material fall off, and thus, the voids cannot be accurately evaluated. For this reason, it is preferred to polish the cold storage material by using an ion milling apparatus which is less likely to cause them to fall off.

The cross-section of the exposed sample is observed by using an optical microscope or an scanning electron microscope (SEM). At this time, it is preferred to specify the voids after excluding dust adhering to the measurement surface by comparing the backscattered electron image and the secondary electron image of the SEM with each other.

As the magnetic cold storage material particles to be evaluated, it is preferred to select the particles, cross-sectional equivalent circle diameter of which is within ±10% of the median value of the particle size distribution, and to observe the voids. A particle whose cross-sectional equivalent circle diameter is outside the range of ±10% from the median value of the particle size distribution means that the cross-section is outside the center of this particle, and there is a possibility that the proportion of voids existing in this particle cannot be accurately evaluated.

The particle size distribution of the magnetic cold storage material particles can be measured by evaluating the distribution of diameters of the perfect circles that correspond to the area of the figure to be observed in an image such as an optical microscope image of the magnetic cold storage material particles and a scanning electron microscope image (SEM image) of the same. In evaluation of the distribution of diameters, it is preferred to evaluate the diameters of 50 or more particles, for example.

Detection of the elements contained in the magnetic cold storage material particles of the first embodiment and measurement of the atomic concentration of the elements can be performed by: dissolving the magnetic cold storage material particles in a liquid; and using Inductively Coupled Plasma Atomic Emission Spectroscopy (ICP-AES), for example. It can also be performed by using energy dispersive X-ray spectroscopy (EDX) or wavelength dispersive X-ray spectroscopy (WDX) .

In addition, the particle size of each magnetic cold storage material particle according to the first embodiment is 50 um or more and 3 mm or less. The aspect ratio of each magnetic cold storage material particle is, for example, 1 or more and 5 or less. The aspect ratio of each magnetic cold storage material particle is the ratio of the long diameter to the short diameter of the magnetic cold storage material particle. The shape of each magnetic cold storage material particle is spherical, for example.

The particle size of the magnetic cold storage material particle is the equivalent circle diameter. The equivalent circle diameter is the diameter of the perfect circle that corresponds to the area of a figure to be observed in an image such as an optical microscope image of the magnetic cold storage material particles and a scanning electron microscope image (SEM image) of the same. The particle size of the magnetic cold storage material particle can be determined by image analysis of the optical microscope image or the SEM image, for example.

The manufacturing method of the magnetic cold storage material particles according to the first embodiment is not particularly limited, and various manufacturing methods can be applied. For example, they can be manufactured by a method in which molten metal of a base alloy having a predetermined composition is rapidly cooled and solidified by a centrifugal atomization method, a plasma rotating electrode method, a gas atomizing method, a rotating electrode, or the like so as to be granulated.

The base alloy can be manufactured by: making a raw material into a molten metal under a high-frequency melting method or the like; and pouring it into a mold. The base alloy can also be manufactured by mixing raw material powders to prepare a raw material mixture; molding the obtained raw material mixture; and sintering it in a vacuum furnace, for example. Further, the magnetic cold storage material particles with an aspect ratio of 1 or more and 5 or less can be obtained by optimizing manufacturing conditions or performing shape classification such as the tilt vibration method, for example.

Under the definition that: the peripheral length of the projected image of the magnetic cold storage material particle is defined as L; and the actual area of the projected image is defined as A, the circularity R represented by 4πA/L² can be a value exceeding 0.4 by optimizing the manufacturing conditions and/or performing the shape classification such as the tilt vibration method.

The magnetic cold storage material particles according to the first embodiment can be manufactured by subjecting the obtained magnetic cold storage material particles to pressure heat treatment. In the pressure heat treatment, the voids can be controlled by controlling the pressure to be applied to the sample and the heat treatment temperature. When a HIP method is used in the pressure heat treatment, the voids can be controlled by controlling the gas pressure and the sintering temperature. The HIP treatment is performed in, for example, a pressurized atmosphere of an inert gas. Argon or nitrogen can be used as the inert gas. The gas pressure is, for example, 1 MPa or more and 200 MPa or less. The heat treatment temperature is, for example, 500°C or higher and 2000°C or lower. The heat treatment temperature is adjusted depending on the melting point of the sample. The heat treatment time is, for example, 1 hour or more and 48 hours or less.

Next, the effects of the magnetic cold storage material particles according to the first embodiment will be described.

In a cryogenic refrigerator, work of filling a cold storage device with cold storage material particles is performed at a room temperature in general. Accordingly, even under the case where the cold storage device is sufficiently filled with them at a room temperature, if it is subjected to an extremely low temperature environment, the gaps between the cold storage material particles or between the cold storage material particles and the cold storage device increase due to thermal shrinkage, and thereby its filling property (fillability) changes. The enlarged gaps between the cold storage material particles cause the cold storage material particles to move inside the cold storage device, and consequently cause rattling such as particle collision in some cases. When the refrigerator is operated in a rattling state, vibration during operation and the reciprocating motion of the working gas inside the cold storage device cause wear (abrasion) and cracking of the cold storage material particles, which leads to reduction in its refrigeration performance.

The smaller the thermal expansion coefficient of the cold storage material particles is, the more it suppresses occurrence of voids due to the thermal shrinkage between the cold storage material particles in a cryogenic environment, the more stable the filling state of the cold storage material particles in the cold storage device becomes, and the more it reduces occurrence frequency of rattling. This reduces the occurrence frequency of wear and cracking of the cold storage material particles during operation of the refrigerator and can stabilize the refrigeration performance of the refrigerator for a long period of time.

The area percentage of voids present in the magnetic cold storage material particles of the embodiment is 0.0001% or more, is preferably 0.0005% or more, and is more preferably 0.001% or more. When the area percentage of voids is 0.0001% or more, the voids present in the particles absorb the shrinkage of the particles, the thermal shrinkage of the cold storage material particles becomes smaller than the thermal shrinkage of the cold storage material particles for which the area percentage of the voids is less than 0.0001%, change in fillability under a cryogenic environment is suppressed, and thereby the occurrence frequency of rattling is reduced. As a result, even if the refrigerator is operated for a long time, the occurrence frequency of wear and cracking of the magnetic cold storage material particles is reduced, and the refrigeration performance can be maintained.

The area percentage of voids present in the magnetic cold storage material particles of the embodiments is 15% or less, and is preferably 10% or less. As the amount of voids present in the magnetic cold storage material particles increases, the strength of the magnetic cold storage material particles tends to be reduced. However, when the area percentage of voids is 15% or less, rattling due to change in fillability is reduced, so the breakage amount of the magnetic cold storage material particles caused by vibration attributable to the operation of the refrigerator is reduced, and consequently, the refrigeration performance can be maintained for a long period of time.

The particle size of the magnetic cold storage material particle of the first embodiment is preferably 50 um or more and 3 mm or less, is more preferably 1 mm or less, and is further preferably 500 µm or less. When the particle size of the magnetic cold storage material particle is 50 um or more, the filling density of the magnetic cold storage material particles in the cold storage device is reduced, the pressure loss of the working medium such as helium is reduced, and consequently, the refrigeration performance of the refrigerator is improved. When the particle diameter of the magnetic cold storage material particle is 3 mm or less, the distance from the surface of the magnetic cold storage material particle to its particle center becomes shorter, and the heat transfer between the working medium and the magnetic cold storage material particles becomes easier to reach the center of the cold storage material, resulting in improvement in the refrigeration performance of the refrigerator.

The aspect ratio of the magnetic cold storage material particle of the embodiment is preferably 5 or less, and is more preferably 2 or less. When the magnetic cold storage material particles have an aspect ratio of 5 or less, the gaps to be generated by filling the cold storage device with the magnetic cold storage material particles become uniform, and thereby the refrigeration performance of the refrigerator is improved.

The circularity R of the magnetic cold storage material particle of the embodiment is preferably larger than 0.4, and is more preferably 0.6 or more. When the circularity R of the magnetic cold storage material particle approaches 1, the shape becomes closer to a sphere, the gaps to be generated by filling the cold storage device with the magnetic cold storage material particles becomes uniform, and thereby its fillability is stabilized, resulting in improvement in the refrigeration performance of the refrigerator.

### (Second Embodiment)

The cold storage device according to the second embodiment is filled with single or plural types of plural cold storage material particles. The cold storage device of the second embodiment is filled with a plurality of magnetic cold storage material particles represented by the above-described "RMz", and 70% or more of these magnetic cold storage material particles represented by RMz are the magnetic cold storage material particles of the first embodiment. For example, under the definition that: the peripheral length of the projected image of the magnetic cold storage material particles of the first embodiment filling the cold storage device is defined as L; the actual area of the projected image is defined as A; and the circularity R is defined as 4πA/L², in the cold storage device according to the second embodiment, the proportion of the magnetic cold storage material particles with a circularity R of 0.4 or less is 15% or less.

When the magnetic cold storage material particles of the first embodiment occupy less than 70% of the magnetic cold storage material particles that fill the cold storage device of the second embodiment and are represented by RMz, the contribution of the magnetic cold storage material particles of the first embodiment is reduced, and thus, it is not possible to reduce the occurrence frequency of rattling under a cryogenic environment. The proportion of the magnetic cold storage material particles of the first embodiment is 70% or more, is preferably 80% or more, is more preferably 90% or more, and is even more preferably 100%. The proportion of the magnetic cold storage material particles of the first embodiment included in the cold storage device can be determined by: extracting a certain number of particles from among the magnetic cold storage material particles represented by RMz and included in the cold storage device; observing the cross-sections of those particles; and calculating the proportion of the particles with an area percentage of voids of 0.0001% or more and 15% or less. At this time, the average value of the area percentage of voids in the particles having an area percentage of voids of 0.0001% or more and 15% or less is the area percentage of voids in the magnetic cold storage material particles of the first embodiment provided in the above-described cold storage device. In the case of evaluating the proportion of the magnetic cold storage material particles of the first embodiment, it is preferred to observe and evaluate cross-sections of ten or more particles, for example.

The circularity R of the magnetic cold storage material particles can be determined by performing image processing on the shapes of the plurality of magnetic cold storage material particles with the use of an optical microscope. The magnetic cold storage material particles with a circularity R of 0.4 or less exhibit a shape including convex and concave portions on their surfaces. If such magnetic cold storage material particles occupy more than 15% of the magnetic cold storage material particles represented by RMz and included in the cold storage device, even in the case of using the magnetic cold storage material particles of the first embodiment, the gaps formed by the magnetic cold storage material particles in the cold storage device become non-ununiform and the fillability becomes unstable, which causes rattling under a cryogenic temperature and reduces the refrigeration performance and the long-term reliability of the refrigerator during long-term operation. The proportion of the magnetic cold storage material particles with a circularity R of 0.4 or less is preferably 15% or less, is more preferably 10% or less, is more preferably 5% or less, is more preferably 2% or less, and is even more preferably 0%. In the case of evaluating the proportion of the magnetic cold storage material particles with a circularity R of 0.4 or less, it is preferred to evaluate 50 or more particles, for example.

### (Third Embodiment)

The refrigerator according to the third embodiment is provided with the cold storage device according to the second embodiment that is filled with the plurality of magnetic cold storage material particles according to the first embodiment. In the following, description of components common to the first or second embodiment is partially omitted.

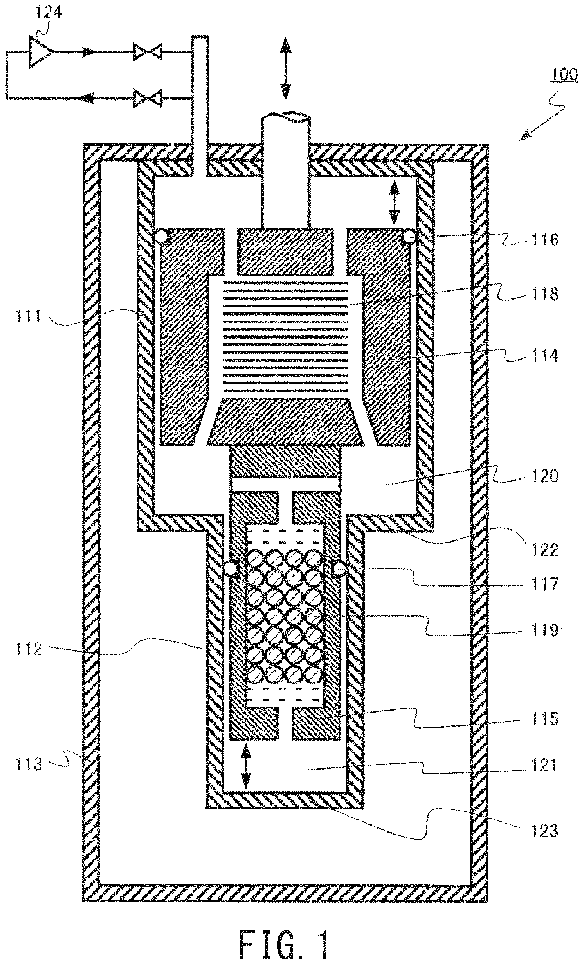

Fig. 1 is a schematic cross-sectional view illustrating the main configuration of the refrigerator according to the third embodiment provided with the cold storage device according to the second embodiment, which is filled with the plurality of magnetic cold storage material particles according to the first embodiment. The refrigerator according to the third embodiment is a two-stage cold storage type cryogenic refrigerator 100 to be used for cooling a superconducting apparatus and the like.

The cold storage type cryogenic refrigerator 100 includes: a first cylinder 111; a second cylinder 112; a vacuum container 113; a first cold storage device 114; a second cold storage device 115; a first seal ring 116; a second seal ring 117; a first cold storage material 118; a second cold storage material 119; a first expansion chamber 120; a second expansion chamber 121; a first cooling stage 122; a second cooling stage 123; and a compressor 124.

The cold storage type cryogenic refrigerator 100 includes the vacuum container 113 in which the large-diameter first cylinder 111 and the small-diameter second cylinder 112 coaxially connected to the first cylinder 111 are installed. The first cold storage device 114 is disposed in the first cylinder 111 so as to be reciprocable. In the second cylinder 112, the second cold storage device 115, which is one aspect of the cold storage device according to the second embodiment, is disposed so as to be reciprocable.

The first seal ring 116 is disposed between the first cylinder 111 and the first cold storage device 114. The second seal ring 117 is disposed between the second cylinder 112 and the second cold storage device 115.

The first cold storage device 114 accommodates the first cold storage material 118 such as a Cu mesh. The second cold storage device 115 accommodates the second cold storage material 119.

Each of the first cold storage device 114 and the second cold storage device 115 has a working-medium passage provided in gaps of the first cold storage material 118 and the second cold storage material 119 and the like. The working medium is helium gas.

The first expansion chamber 120 is provided between the first cold storage device 114 and the second cold storage device 115. The second expansion chamber 121 is provided between the second cold storage device 115 and a distal end wall of the second cylinder 112. The first cooling stage 122 is provided at the bottom of the first expansion chamber 120. The second cooling stage 123 lower in temperature than the first cooling stage 122 is formed at the bottom of the second expansion chamber 121.

A high-pressure working medium is supplied from the compressor 124 to the above-described two-stage cold storage type cryogenic refrigerator 100. The supplied working medium passes through the first cold storage material 118 accommodated in the first cold storage device 114 so as to reach the first expansion chamber 120, and then passes through the second cold storage material 119 accommodated in the second cold storage device 115 so as to reach the second expansion chamber 121.

At this time, the working medium is cooled by supplying thermal energy to the first cold storage material 118 and the second cold storage material 119. The working medium having passed through the first cold storage material 118 and the second cold storage material 119 expands in the first expansion chamber 120 and the second expansion chamber 121 so as to generate cold. Consequently, the first cooling stage 122 and the second cooling stage 123 are cooled down.

The expanded working medium flows in the opposite direction between the first cold storage material 118 and the second cold storage material 119. The working medium is discharged after receiving thermal energy from the first cold storage material 118 and the second cold storage material 119. The cold storage type cryogenic refrigerator 100 is configured in such a manner that: the thermal efficiency of the working-medium cycle is improved as the heat recuperation effect becomes satisfactory in the course of the above-described process; and thereby a lower temperature is realized.

The second cold storage device 115 is the cold storage device according to the second embodiment, and the cold storage device provided in the refrigerator according to the third embodiment accommodates the plurality of magnetic cold storage material particles according to the first embodiment as the second cold storage material 119 at least as a part. Under the definition that: the peripheral length of the projected image of each of the magnetic cold storage material particles is defined as L; the actual area of the projected image is defined as A; and the circularity R is defined as 4πA/L², in the plurality of magnetic cold storage material particles according to the first embodiment, the proportion of particles having a circularity R of 0.4 or less is preferably 15% or less.

In the third embodiment, the cold storage device according to the second embodiment may include a plurality of cold-storage-material filling layers of different types of cold storage material, for example. The different types of cold storage material may be separated by a mesh. The mesh is, for example, a metal mesh. At least one of the plurality of cold-storage-material filling layers is the magnetic cold storage material particles according to the first embodiment. In the refrigerator of the third embodiment, when the magnetic cold storage material particles of the first embodiment are combined with the cold storage material particles that is lower in specific-heat maximum-value than the magnetic cold storage material particles of the first embodiment, the plurality of magnetic cold storage material particles of the first embodiment are used for filling the high-temperature side of the cold storage device, for example.

When the magnetic cold storage material particles of the first embodiment are combined with the cold storage material particles that is higher in specific-heat maximum-value than the magnetic cold storage material particles of the first embodiment, the plurality of magnetic cold storage material particles of the first embodiment are used for filling the low-temperature side of the cold storage device, for example.

When the magnetic cold storage material particles of the first embodiment are combined with both types of: the cold storage material particles higher in specific-heat maximum-value than the magnetic cold storage material particles of the first embodiment; and the cold storage material particles lower in specific-heat maximum-value than the magnetic cold storage material particles of the first embodiment, the plurality of magnetic cold storage material particles of the first embodiment are packed in the state of being sandwiched between both types of cold storage material particles in the cold storage device, for example. Even when the magnetic cold storage material particles of the first embodiment are used and particles of different compositions are combined, depending on the temperature at which the specific heat peaks, it can be determined whether it is the cold side or the hot side of the cold storage device.

In order to improve the reliability of the refrigerator, it is preferred that the breakage amount of the cold storage material particles in the case of being subjected to vibration for a long time under a cryogenic environment is equal to or below a certain level. The refrigerator of the third embodiment includes the cold storage device of the second embodiment that is provided with the magnetic cold storage material particles of the first embodiment.

The long-term reliability of a magnetically levitated train and a helium re-condensation apparatus can be improved by using the refrigerator of the third embodiment for the magnetically levitated train and the helium re-condensation apparatus.

According to the third embodiment, a refrigerator with excellent long-term reliability can be achieved by using the magnetic cold storage material particles with excellent characteristics including the reduced breakage amount.

### (Fourth Embodiment)

The cryopump according to the fourth embodiment includes the refrigerator according to the third embodiment. In the following, description of components common to the third embodiment is partially omitted.

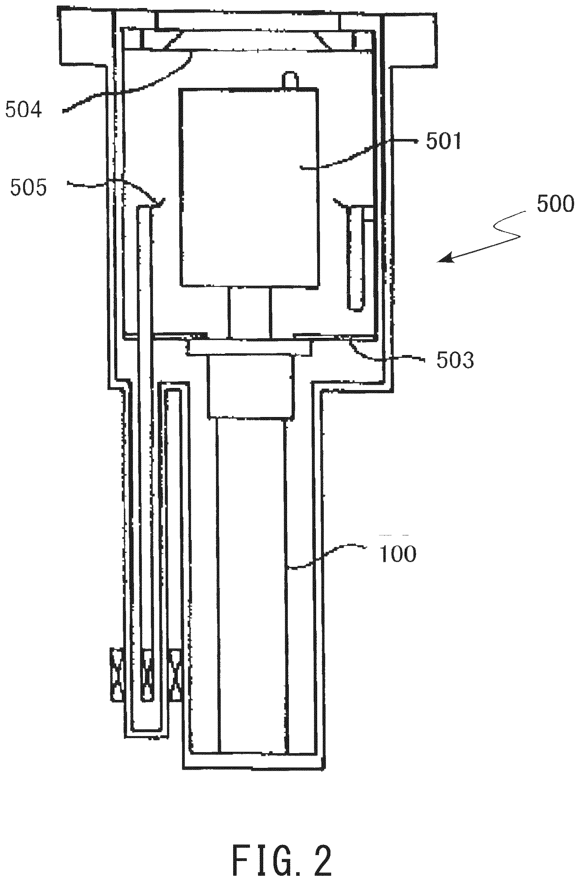

Fig. 2 is a cross-sectional view illustrating an overall configuration of the cryopump according to the fourth embodiment. The cryopump 500 according to the fourth embodiment includes the cold storage type cryogenic refrigerator 100 according to the third embodiment.

The cryopump 500 includes: a cryopanel 501 that condenses or adsorbs gas molecules; the cold storage type cryogenic refrigerator 100 that cools down the cryopanel 501 to a predetermined cryogenic temperature; a shield 503 provided between the cryopanel 501 and the cold storage type cryogenic refrigerator 100; a baffle 504 provided at an intake port; and a ring 505 that changes exhaust speed of argon, nitrogen, hydrogen, or the like.

According to the fourth embodiment, a cryopump having excellent long-term reliability can be achieved by using the refrigerator that is excellent in terms of long-term reliability.

### (Fifth Embodiment)

The superconducting magnet according to the fifth embodiment includes the refrigerator according to the third embodiment. In the following, description of components common to the third embodiment is partially omitted.

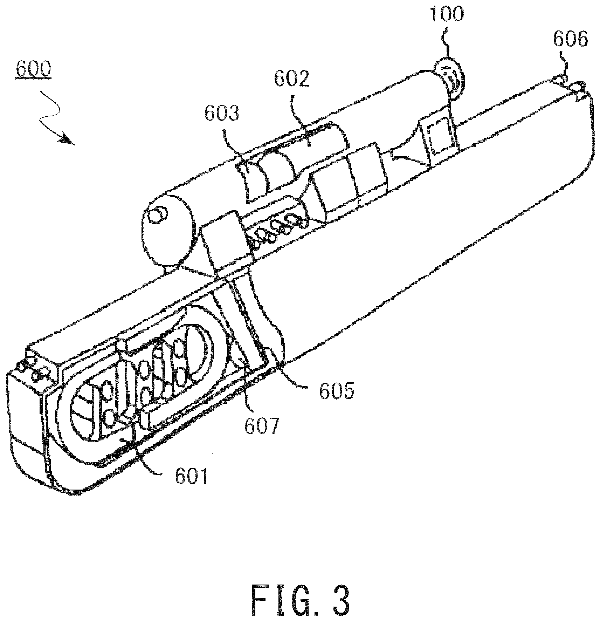

Fig. 3 is a perspective view illustrating an overall configuration of the superconducting magnet according to the fifth embodiment. The superconducting magnet according to the fifth embodiment is a superconducting magnet 600 for a magnetically levitated train including the cold storage type cryogenic refrigerator 100 according to the third embodiment.

The superconducting magnet 600 for a magnetically levitated train includes: a superconducting coil 601; a liquid helium tank 602 for cooling down the superconducting coil 601; a liquid nitrogen tank 603 for preventing volatilization of liquid helium; a laminated heat insulating material 605; a power lead 606; a permanent current switch 607; and the cold storage type cryogenic refrigerator 100.

According to the fifth embodiment, a superconducting magnet having excellent long-term reliability can be achieved by using the refrigerator having excellent long-term reliability.

### (Sixth Embodiment)

The MRI apparatus according to the sixth embodiment includes the refrigerator according to the third embodiment. In the following, description of components common to the third embodiment is partially omitted.

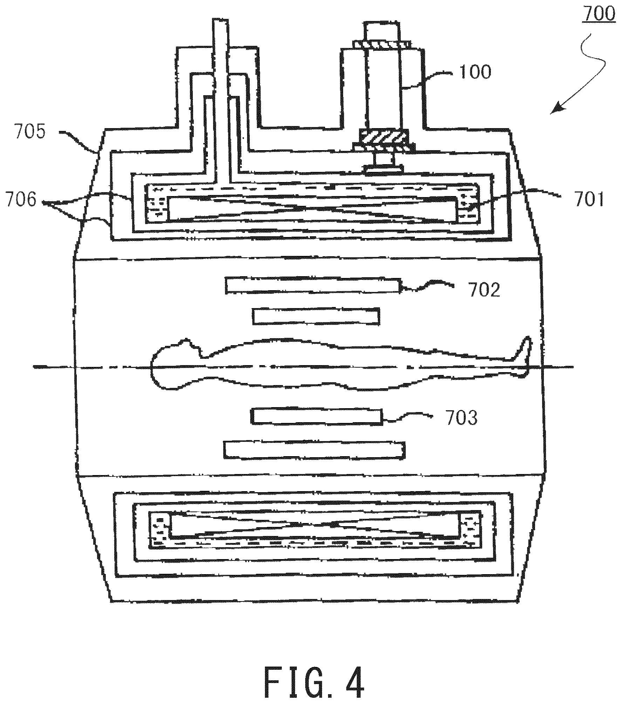

Fig. 4 is a cross-sectional view illustrating an overall configuration of the MRI apparatus according to the sixth embodiment. The MRI apparatus 700 according to the sixth embodiment includes the cold storage type cryogenic refrigerator 100 according to the third embodiment.

The MRI apparatus 700 includes: a superconducting static magnetic field coil 701 that applies a spatially uniform and temporally stable static magnetic field to a human body; a correction coil (not shown) that corrects non-uniformity of the generated magnetic field; a gradient magnetic field coil 702 that gives a magnetic field gradient to a measurement region; a radio-wave transmission/reception probe 703; a cryostat 705; and a radiation adiabatic shield 706. The cold storage type cryogenic refrigerator 100 is used for cooling down the superconducting static magnetic field coil 701.

According to the sixth embodiment, an MRI apparatus having excellent long-term reliability can be achieved by using the refrigerator having excellent long-term reliability.

### (Seventh Embodiment)

The nuclear magnetic resonance apparatus according to the seventh embodiment includes the refrigerator according to the third embodiment. In the following, description of components common to the third embodiment is partially omitted.

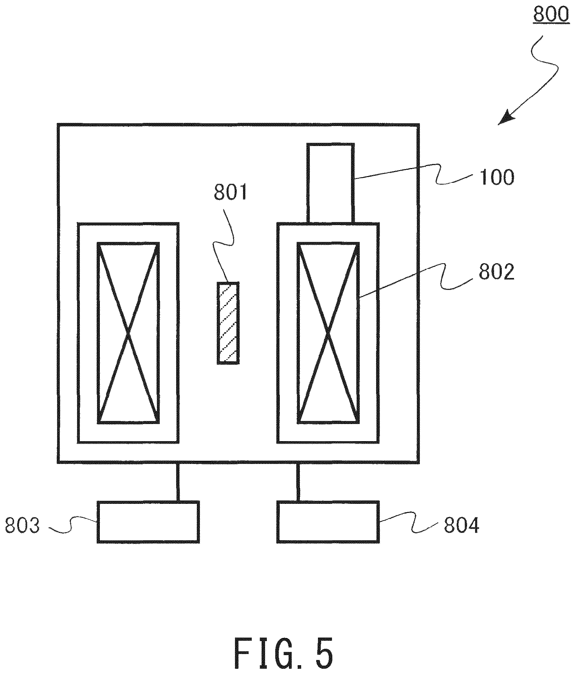

Fig. 5 is a cross-sectional view illustrating an overall configuration of the nuclear magnetic resonance apparatus according to the seventh embodiment. The nuclear magnetic resonance (NMR) apparatus 800 according to the seventh embodiment includes the cold storage type cryogenic refrigerator 100 according to the third embodiment.

The nuclear magnetic resonance apparatus 800 includes: a superconducting static magnetic field coil 802 that applies a magnetic field to a sample such as an organic substance placed in a sample tube 801; a high frequency oscillator 803 that applies a radio wave to the sample tube 801 in the magnetic field; and an amplifier 804 that amplifies an induced current to be generated in a coil (not shown) around the sample tube 801. In addition, the cold storage type cryogenic refrigerator 100 for cooling down the superconducting static magnetic field coil 802 is provided.

According to the seventh embodiment, a nuclear magnetic resonance apparatus having excellent long-term reliability can be achieved by using the refrigerator having excellent long-term reliability.

### (Eighth Embodiment)

The magnetic-field-application-type single-crystal puller according to the eighth embodiment includes the refrigerator according to the third embodiment. In the following, description of components common to the third embodiment is partially omitted.

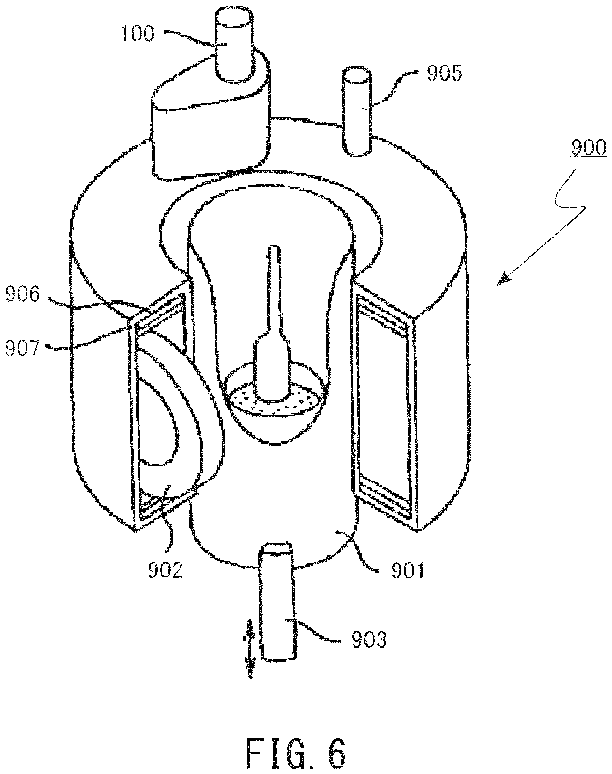

Fig. 6 is a perspective view illustrating an overall configuration of the magnetic-field-application-type single-crystal puller according to the eighth embodiment. The magnetic-field-application-type single-crystal puller 900 according to the eighth embodiment includes the cold storage type cryogenic refrigerator 100 according to the third embodiment.

The magnetic-field-application-type single-crystal puller 900 includes: a single crystal pulling unit 901 provided with a raw material melting crucible, a heater, a single crystal pulling mechanism, and the like; a superconducting coil 902 that applies a static magnetic field to the raw material melt; a lifting/lowering mechanism 903 for the single crystal pulling unit 901; a current lead 905; a heat shield plate 906; and a helium container 907. The cold storage type cryogenic refrigerator 100 is used for cooling down the superconducting coil 902.

According to the eighth embodiment, a magnetic-field-application-type single-crystal puller having excellent long-term reliability can be achieved by using the refrigerator having excellent long-term reliability.

### (Ninth Embodiment)

The helium re-condensation apparatus according to the ninth embodiment includes the refrigerator according to the third embodiment. In the following, description of components common to the third embodiment is partially omitted.

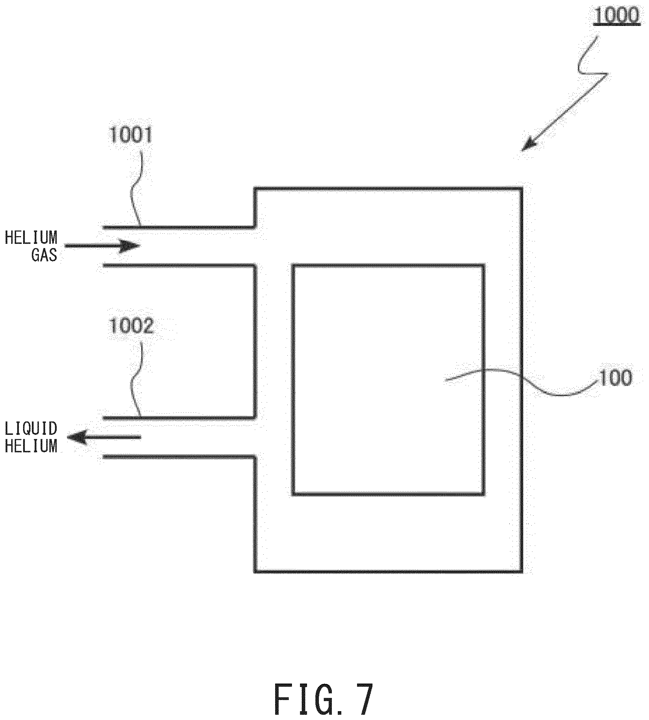

Fig. 7 is a schematic diagram illustrating an overall configuration of the helium re-condensation apparatus according to the ninth embodiment. The helium re-condensation apparatus 1000 according to the ninth embodiment includes the cold storage type cryogenic refrigerator 100 according to the third embodiment.

The helium re-condensation apparatus 1000 includes: the cold storage type cryogenic refrigerator 100; an evaporation pipe 1001; and a liquefaction pipe 1002.

The helium re-condensation device 1000 can generate liquid helium by re-condensing helium gas evaporated from: an apparatus that uses liquid helium (e.g., a superconducting magnet, a nuclear magnetic resonance (NMR) apparatus, an MRI apparatus, and a physical property measurement system (PPMS)); or a liquid helium apparatus provided in a device that uses a superconducting magnet, such as a magnetic property measurement system.

The helium gas is introduced into the helium re-condensation apparatus 1000 through the evaporation pipe 1001 from a liquid helium apparatus (not shown). The helium gas is cooled down to 4K below the liquefying temperature of helium by the cold storage type cryogenic refrigerator 100. The condensed liquid helium returns to the liquid helium apparatus through the liquefaction pipe 1002.

According to the ninth embodiment, a helium re-condensation apparatus having excellent long-term reliability can be achieved by using the refrigerator having excellent long-term reliability.

The refrigerator according to the third embodiment can improve long-term reliability by being used for a magnetically levitated train.

### [Examples]

Hereinbelow, a description will be given of examples of the magnetic cold storage material particles according to the first embodiment, comparative examples, and their evaluation results.

### (Example 1)

An Er₃Ni mother alloy was produced by high-frequency melting. This Er₃Ni mother alloy was melted at about 800°C, and this molten metal was dropped onto a rotating disk in an Ar atmosphere (at a pressure of about 101 kPa) so as to be rapidly cooled and solidified. The resultant grains were subjected to shape classification and sieving, and thereby Er₃Ni spherical particles were obtained.

The Er₃Ni spherical particles were subjected to the HIP treatment. At this time, nitrogen gas was used as the pressurized gas. The pressure was 50 MPa and the heat treatment temperature was 600°C.

The Er₃Ni spherical particles of Example 1 have an aspect ratio of 1.1. The area percentage of voids is 0.0012%.

In order to evaluate the reliability when the cold storage device is filled with the magnetic cold storage material particles and the refrigerator is operated, a cylindrical container with a diameter of 15 mm and a height of 5 mm was filled with the magnetic cold storage material particles of Example 1 at a room temperature, and a single vibration with an amplitude of 2 mm and a maximum acceleration of 400 m/s² was applied to the container 2×10⁹ times under the state where the container was cooled down to a cryogenic temperature. The weight percentage of the cold storage material particles destroyed by the vibration test was 0.014%.

For the cold storage material of Example 1, its refrigeration capacity at 4.2K in the case of being mounted on the refrigerator was evaluated before and after the above-described vibration test. The cold storage device was filled with them at a room temperature to produce a cryogenic cold storage device. A two-stage GM refrigerator was used for the refrigerator, a Cu mesh was used for the first-stage cold storage device, Pb was used for the high-temperature side of the second-stage cold storage device, and a cold storage device filled with the cold storage material of each of examples and comparative examples was used for the low-temperature side of the second-stage cold storage device. The cold storage material particles after the vibration test were mounted including the cold storage material particles destroyed by the vibration test, and a heat load was applied to the first-stage cold storage device such that the temperature was 50K.

In the following examples and comparative examples, the compound conditions of magnetic cold storage material particles and the HIP treatment conditions were adjusted to be appropriate conditions. The vibration test at a cryogenic temperature and the capacity test of the refrigerator were performed under the same conditions as in Example 1.

### (Example 2)

Magnetic cold storage material particles were produced in the same manner as in Example 1 except that the composition was Er₃Co.

### (Example 3)

Magnetic cold storage material particles were produced in the same manner as in Example 1 except that the composition was ErAg.

### (Example 4)

Magnetic cold storage material particles were produced in the same manner as in Example 1 except that the composition was ErNi.

### (Example 5)

Magnetic cold storage material particles were produced in the same manner as in Example 1 except that the composition was HoCu₂.

### (Example 6)

Magnetic cold storage material particles were produced in the same manner as in Example 1 except that the composition was HoCu.

### (Example 7)

Magnetic cold storage material particles were produced in the same manner as in Example 1 except that the composition was Ho₂Al.

### (Example 8)

Magnetic cold storage material particles were produced in the same manner as in Example 1 except that the composition was Dy(Cu_{0.5}Si_{0.5})₂.

### (Example 9)

Magnetic cold storage material particles were produced in the same manner as in Example 1 except that the composition was Dy(Cu_{0.5}Ge_{0.5})₂.

### (Examples 10 to 14)

Magnetic cold storage material particles were produced in the same manner as in Example 1 except that the gas pressure in the HIP treatment was changed.

### (Comparative Example 1)

Magnetic cold storage material particles were produced in the same manner as in Example 1 except that the pressure in the HIP treatment was 190 MPa.

### (Comparative Example 2)

Magnetic cold storage material particles were produced in the same manner as in Example 1 except that the pressure in the HIP treatment was 10 MPa.

### (Examples 15 to 20)

Magnetic cold storage material particles were produced in the same manner as in Example 5 except that the shape-classification conditions such as the tilt angle and the vibration strength were adjusted.

### (Examples 21 to 24 and Comparative Example 3)

The magnetic cold storage material particles of Example 1 and Comparative Example 3 were used as the cold storage material particles for filling cold storage devices, and vibration tests and refrigerator tests were performed by using the cold storage devices with different proportions of them.

### (Examples 25 to 27 and Comparative Example 4)

The vibration tests and refrigerator tests were performed by using the cold storage devices with different proportions of both: the magnetic cold storage material particles of Example 5; and the magnetic cold storage material particles that were produced in the same manner as in Example 5 except adjusted shape-classification conditions such as the tilt angle and had the same properties as Example 5 except inclusion of particles with a circularity of 0.4 or less.

Table 1 shows the weight percentage of broken particles after the vibration test and the refrigeration capacity of the magnetic cold storage material particles for each of examples and comparative examples.

**Table 1**

| | METALLIC MAGNETIC COLD STORAGE MATERIAL | AREA PERCENTAGE OF VOIDS | PROPORTION OF PARTICLES, IN EACH OF WHICH AREA PERCENTAGE OF VOIDS IS 0.0001% OR MORE AND 15% OR LESS | PROPORTION OF COLD STORAGE MATERIAL PARTICLES WITH CIRCULARITY OF 0.4 OR LESS | PARTICLE SIZE (µm) OF COLD STORAGE MATERIAL PARTICLES | AVERAGE ASPECT RATIO OF COLD STORAGE MATERIAL PARTICLES | WEIGHT PERCENTAGE (%) OF PARTICLES BROKEN AFTER VIBRATION TEST AT CRYOGENIC TEMPERATURE | REFRIGE -RATION CAPACITY (W) AT 4.2K | RATIO OF REFRIGE -RATING CAPACITY BEFORE AND AFTER VIBRATION TEST |
|---|---|---|---|---|---|---|---|---|---|
| EXAMPLE 1 | Er₃Ni | 0.0012% | 100% | 0% | 250 | 1.1 | 0.014 | - | 100% |
| EXAMPLE 2 | Er₃Co | 0.015% | 100% | 0% | 400 | 1 | 0.014 | - | 100% |
| EXAMPLE 3 | ErAg | 0.031% | 100% | 0% | 260 | 1 | 0.014 | - | 100% |
| EXAMPLE 4 | ErNi | 0.028% | 100% | 0% | 440 | 1.1 | 0.014 | - | 100% |
| EXAMPLE 5 | HoCu₂ | 0.008% | 100% | 0% | 220 | 1 | 0.014 | - | 100% |
| EXAMPLE 6 | HoCu | 0.500% | 100% | 0% | 242 | 1.1 | 0.04 | - | 99% |
| EXAMPLE 7 | Ho₂Al | 0.710% | 100% | 0% | 360 | 1 | 0.04 | - | 99% |
| EXAMPLE 8 | Dy(Cu_{0.5}Si_{0.5})₂ | 1.300% | 100% | 0% | 550 | 1.4 | 0.04 | - | 99% |
| EXAMPLE 9 | Dy(Cu_{0.5}Ge_{0.5})₂ | 2.100% | 100% | 0% | 1000 | 1.6 | 0.05 | - | 99% |
| EXAMPLE 10 | Er₃Ni | 0.0002% | 100% | 0% | 251 | 1 | 0.9 | - | 91% |
| EXAMPLE 11 | Er₃Ni | 0.12% | 100% | 0% | 250 | 1 | 0.01 | - | 100% |
| EXAMPLE 12 | Er₃Ni | 1.20% | 100% | 0% | 250 | 1 | 0.09 | - | 99% |
| EXAMPLE 13 | Er₃Ni | 5.10% | 100% | 0% | 260 | 1 | 0.2 | - | 95% |
| EXAMPLE 14 | Er₃Ni | 9.50% | 100% | 0% | 250 | 1 | 0.9 | - | 91% |
| EXAMPLE 15 | HoCu₂ | 0.008% | 100% | 0% | 50 | 1 | 0.01 | 0.80 | 100% |
| EXAMPLE 16 | HoCu₂ | 0.008% | 100% | 0% | 3000 | 1 | 0.01 | 0.78 | 100% |
| EXAMPLE 17 | HoCu₂ | 0.008% | 100% | 0% | 40 | 1 | 0.4 | 0.58 | 94% |
| EXAMPLE 18 | HoCu₂ | 0.008% | 100% | 0% | 4000 | 1 | 0.4 | 0.60 | 94% |
| EXAMPLE 19 | HoCu₂ | 0.008% | 100% | 0% | 250 | 4.8 | 0.05 | 0.75 | 99% |
| EXAMPLE 20 | HoCu₂ | 0.008% | 100% | 0% | 250 | 5.5 | 0.5 | 0.59 | 95% |
| EXAMPLE 21 | Er₃Ni | 0.0012% | 100% | 0% | 250 | 1 | 0.01 | - | 100% |
| EXAMPLE 22 | Er₃Ni | 0.0012% | 93% | 0% | 250 | 1 | 0.05 | - | 97% |
| EXAMPLE 23 | Er₃Ni | 0.0012% | 81% | 0% | 250 | 1 | 0.5 | - | 95% |
| EXAMPLE 24 | Er₃Ni | 0.0012% | 72% | 0% | 250 | 1 | 0.8 | - | 92% |
| EXAMPLE 25 | HoCu₂ | 0.008% | 100% | 1% | 220 | - | 0.01 | - | 99% |
| EXAMPLE 26 | HoCu₂ | 0.008% | 100% | 5% | 220 | - | 0.05 | - | 97% |
| EXAMPLE 27 | HoCu₂ | 0.008% | 100% | 9% | 220 | - | 0.5 | - | 95% |
| COMPARATIVE EXAMPLE 1 | Er₃Ni | 0.00009% | 100% | 0% | 250 | 1 | 1.2 | - | 80% |
| COMPARATIVE EXAMPLE 2 | Er₃Ni | 16.000% | 100% | 0% | 250 | 1 | 1.2 | - | 79% |
| COMPARATIVE EXAMPLE 3 | Er₃Ni | 0.009% | 60% | 0% | 440 | 1.1 | 1.2 | - | 81% |
| COMPARATIVE EXAMPLE 4 | HoCu₂ | 0.008% | 100% | 11% | 220 | 1.2 | 1.5 | - | 70% |

As shown in Examples 1 to 27 and Comparative Example 1, when the area percentage of voids in the cross-section of the magnetic cold storage material particle is 0.0001% or more, the proportion of particles destroyed by the vibration test is reduced, and the rate of change in the refrigeration capacity of the refrigerator also decreases. This is presumably because the presence of more than a certain amount of voids suppresses the thermal shrinkage of the particles and reduces rattling due to change in fillability of the particles to be caused by the shrinkage in association with cooling.

As shown in Examples 10 to 14 and Comparative Example 2, when the area percentage of voids in the cross-section of the magnetic cold storage material particle is less than 15%, the proportion of the particles destroyed by the vibration test decreases and the rate of change in the refrigeration capacity of the refrigerator also decreases. This is presumably because reducing the voids below a certain amount suppresses the thermal shrinkage of the particles and maintains the strength of the magnetic cold storage material particles.

As is clear from Table 1, when the particle size of the magnetic cold storage material particles falls within the range from 50 um to 3000 um, the refrigeration capacity at 4.2K is significantly improved.

As is clear from Table 1, when the aspect ratio of the magnetic cold storage material particle becomes 5 or less, the refrigeration capacity at 4.2K is significantly improved.

As is shown in Table 1, when the cold storage material particles with a void area percentage of 0.0001% or more and 15% or less occupy 70% or more of the magnetic cold storage material particles represented by RMz and included in the cold storage device, the proportion of the particles broken by the vibration test is reduced and the decrement in the refrigeration capacity becomes smaller. When the cold storage material particles with a void area percentage of 0.001% or more and 10% or less occupy 70% or more, it is also shown that the proportion of the particles broken by the vibration test is further reduced and the decrement in the refrigeration capacity becomes further smaller.

As is clear from Table 1, when magnetic cold storage material particles with a circularity R of 0.4 or less occupy more than 15% of the magnetic cold storage material particles represented by RMz and included in the cold storage device, the proportion of the particles broken by the vibration test increases and the refrigeration capacity reduces.

As can be seen from Table 1, even when the magnetic cold storage material particles have different compositions, the particle breakage rate in the case of being subjected to vibration at a cryogenic temperatures is low as long as the void percentage is 0.0001% or more and 15% or less.

The above-described examples demonstrate the effects of the magnetic cold storage material particles according to the first embodiment and the cold storage device according to the

### second embodiment.

While certain embodiments have been described, these embodiments have been presented by way of example only, and are not intended to limit the scope of the inventions. Indeed, the novel methods and systems described herein may be embodied in a variety of other forms; furthermore, various omissions, substitutions and changes in the form of the methods and systems described herein may be made without departing from the spirit of the inventions. The accompanying claims and their equivalents are intended to cover such forms or modifications as would fall within the scope and spirit of the inventions.

### REFERENCE SIGNS LIST

- 100: cold storage type cryogenic refrigerator
- 114, 115: cold storage device
- 118, 119: cold storage material
- 500: cryopump
- 600: superconducting magnet
- 700: MRI apparatus
- 800: nuclear magnetic resonance apparatus
- 900: magnetic-field-application-type single-crystal puller

## Claims

1. A magnetic cold storage material particle composed of an intermetallic compound containing a rare earth element represented by RMz, wherein: R is at least one rare earth element selected from Y, La, Ce, Pr, Nd, Pm, Sm, Eu, Gd, Tb, Dy, Ho, Er, Tm and Yb; M is at least one metal element selected from Ni, Co, Cu, Ag, Ga, Bi, Si, Al, and Ru; and z is number in a range from 0.001 to 9.0,
wherein an area percentage of voids present in a cross-section of the magnetic cold storage material particle is 0.0001% or more and 15% or less.

2. The magnetic cold storage material particle according to claim 1, wherein the magnetic cold storage material particle is a grain with a particle size of 50 um or more and 3 mm or less.

3. The magnetic cold storage material particle according to claim 2, wherein an aspect ratio is in a range from 1 to 5.

4. A cold storage device including a plurality of magnetic cold storage material particles represented by the RMz, wherein the magnetic cold storage material particle according to any one of claim 1 to claim 3 occupies 70% or more of the plurality of magnetic cold storage material particles represented by the RMz and included in the cold storage device.

5. The cold storage device according to claim 4, wherein, when a peripheral length of a projected image of the plurality of magnetic cold storage material particles is defined as L; and an actual area of the projected image is defined as A; and a circularity R is defined as 4πA/L², a proportion of magnetic cold storage material particles with a circularity R of 0.4 or less is 15% or less.

6. A refrigerator comprising the cold storage device according to claim 4 or claim 5.

7. A cryopump comprising the refrigerator according to claim 6.

8. A superconducting magnet comprising the refrigerator according to claim 6.

9. A magnetic resonance imaging apparatus comprising the refrigerator according to claim 6.

10. A nuclear magnetic resonance apparatus comprising the refrigerator according to claim 6.

11. A magnetic-field-application-type single-crystal puller comprising the refrigerator according to claim 6.

12. A helium re-condensation apparatus comprising the refrigerator according to claim 6.
